# EUROPÄISCHE PATENTSCHRIFT

(11) **EP 3 820 436 B1**
(45) Veröffentlichungstag und Bekanntmachung des Hinweises auf die Patenterteilung: **08.01.2025**
(21) Anmeldenummer: 19730332.4
(22) Anmeldetag: 12.06.2019
(51) Int. Cl.: A61K 8/49, A61Q 19/00, A61K 8/02, A61Q 19/02

(54) **SELBSTKLEBENDE FLÄCHIGE PRODUKTE ENTHALTEND EIN ODER MEHRERE ALKYLAMIDOTHIAZOLE**
SELF-ADHESIVE FLAT PRODUCTS CONTAINING ONE OR MORE ALKYLAMIDOTHIAZOLES
PRODUITS PLATS AUTO-ADHÉSIFS CONTENANT UN OU PLUSIEURS ALKYLAMIDOTHIAZOLES

(30) Priorität: 10.07.2018 DE 102018211412
(43) Veröffentlichungstag der Anmeldung: 19.05.2021
(73) Patentinhaber: Beiersdorf AG, 22529 Hamburg (DE)
(72) Erfinder: BIERGIESSER, Helga, 21465 Reinbek (DE); FAHRIG, Nele Felicitas, 22305 Hamburg (DE); WOELLER, Karl-Heinz, 20257 Hamburg (DE); STEFFEN-REHKOPF, Sabrina, 22765 Hamburg (DE); BRETSCHNEIDER, Thorsten, 25421 Pinneberg (DE); BREITZKREUTZ, Sabrina, 20251 Hamburg (DE)
(74) Vertreter: Beiersdorf AG
(86) Internationale Anmeldenummer: PCT/EP2019/065291
(87) Internationale Veröffentlichungsnummer: WO 2020/011470

(56) Entgegenhaltungen:
- EP-A1- 1 649 852
- EP-A1- 2 758 381
- EP-B1- 2 758 381
- DE-A1- 102005 053 909
- DE-A1- 102010 012 594

## Beschreibung

Wie in den Ansprüchen definiert, betrifft die vorliegende Erfindung selbstklebende flächige Gebilde, insbesondere kosmetische und / oder medizinische Pflaster, die ein oder mehrere Alkylamidothiazole enthalten in Kombination mit einem Matrixsystem.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozess der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden, dass für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw. - vernarbung (postinflammatorische Hyperpigmentierung) oder der Hautalterung (z.B. *Lentigines seniles*).

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté - Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrundeliegende Entzündung meist subklinisch abläuft.

In vielen Fällen werden derartige postinflammatorische Fehlpigmentierung durch Einwirkung von Sonnenlicht (UV-Licht) noch verstärkt, ohne dass es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im Wesentlichen Präparate auf der Grundlage von Hydrochinon, welche aber einesteils erst nach mehrwöchiger Anwendung ihre Wirkung zeigen, deren übertrieben lange Anwendung, andererseits aus toxikologischen Gründen bedenklich ist. Von Albert Kligman et al. wurde eine sogenannte Triformula entwickelt, die eine Kombination aus 0,1 % Tretinoin, 5,0 % Hydrochinon, 0,1 % Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Transdermale therapeutische Systeme ("TTS") sind an sich bekannt. Nachteilig bei TTS, ist, dass üblicherweise während der Applikationszeit nur ca. 10 % bis 20 % der Wirkstoffbeladung des Pflasters freigesetzt werden. (Kommentar zum Europäischen Arzneibuch, Wissenschaftliche Verlagsgesellschaft Stuttgart, Stand: Aktualisierungslieferung 2009).

Ziel der nachfolgenden Erfindung war es also, den Nachteilen des Standes der Technik Abhilfe zu verschaffen.

Es war überraschend und für den Fachmann nicht vorhersehbar, dass Übelstände des Standes der Technik beseitigt werden durch selbstklebende flächige Gebilde, insbesondere kosmetische und/oder medizinische Pflaster, welche als Wirkstoff ein oder mehrere Alkylamidothiazole enthalten.

Kosmetische Zubereitungen mit einem oder mehreren Alkylamidothiazolen sind bekannt, beispielsweise aus der EP 2 758 381.

Vorteilhafte Alkylamidothiazole im Sinne der vorliegenden Erfindung sind Substanzen der allgemeinen Formel 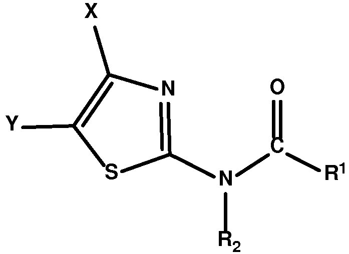 bei welcher
R¹, R², X und Y können unterschiedlich, teilweise gleich oder völlig gleich sein und unabhängig voneinander bedeuten können:
**R₁** = -C₁-C₂₄-Alkyl (linear und verzweigt), -C₁-C₂₄-Alkenyl (linear und verzweigt), -C₁-C₈-Cycloalkyl, -C₁-C₈-Cycloalkyl-Alkylhydroxy, -C₁-C₂₄ Alkylhydroxy (linear und verzweigt), -C₁-C₂₄ Alkylamin (linear und verzweigt), -C₁-C₂₄-Alkylaryl (linear und verzweigt), -C₁-C₂₄-Alkylaryl-Alkyl-Hydroxy (linear und verzweigt), -C₁-C₂₄-Alkylheteroaryl (linear und verzweigt), -C₁-C₂₄-Alkyl-O-C₁-C₂₄-Alkyl (linear und verzweigt), -C₁-C₂₄ Alky-Morpholino, -C₁-C₂₄ Alky-Piperidino, -C₁-C₂₄ Alky-Piperazino, -C₁-C₂₄ Alky-Piperazino-N-Alkyl bedeutet,
**R₂** = H, -C₁-C₂₄-Alkyl (linear und verzweigt), -C₁-C₂₄-Alkenyl (linear und verzweigt), -C₁-C₈-Cycloalkyl, -C₁-C₂₄-Hydroxyalkyl (linear und verzweigt), -C₁-C₂₄-Alkylaryl (linear und verzweigt), -C₁-C₂₄-Alkylheteroaryl (linear und verzweigt), bedeutet,
**X** = -H, -C₁-C₂₄-Alkyl (linear und verzweigt), -C₁-C₂₄-Alkenyl (linear und verzweigt), -C₁-C₈-CycloAlkyl, -C₁-C₂₄-Aryl (ggfs. einfach oder mehrfach substituiert mit -OH, -F, -Cl, -Br, -I, - OMe, -NH₂, -CN) , -C₁-C₂₄-Heteroaryl (ggfs. einfach oder mehrfach substituiert mit -OH, -F, - CI, -Br, -I, -OMe, -NH₂, -CN), -C₁-C₂₄-Alkylaryl (linear und verzweigt), -C₁-C₂₄-Alkylheteroaryl (linear und verzweigt), -Aryl (ggfs. einfach oder mehrfach substituiert mit -OH, -F, -Cl, - Br, -I, -OMe, -NH₂, -CN), -phenyl, -2,4-dihydroxyphenyl, -2,3-dihydroxyphenyl, -2,4-Dimethoxyphenyl, -2,3-Dimethoxyphenyl bedeutet,
**Y** = H, -C₁-C₂₄-Alkyl (linear und verzweigt), -C₁-C₂₄-Alkenyl (linear und verzweigt), -C₁-C₈-Cycloalkyl, -C₁-C₂₄-Aryl, -C₁-C₂₄-Heteroaryl, -C₁-C₂₄-Alkylaryl (linear und verzweigt), -C₁-C₂₄-Alkylheteroaryl (linear und verzweigt), -Aryl, -phenyl, -2,4-dihydroxyphenyl, -2,3-dihydroxyphenyl, -2,4-Dimethoxyphenyl, -2,3-Dimethoxyphenyl, -COO-Alkyl, -COO-Alkenyl, - COO-Cylcloalkyl, -COO-Aryl, -COO-Heteroaryl , bedeutet,
und **X, Y** gegebenenfalls auch = kondensierter Aromat bedeuten können,
wobei **X** und **Y** untereinander aromatische oder aliphatische homo- oder heterozyklische Ringsysteme mit bis n ringbildenden Atomen ausbilden können, und wobei die Zahl n Werte von 5 bis 8 annehmen kann, und die jeweiligen Ringsysteme wiederum mit bis zu n - 1 Alkylgruppen, Hydroxylgruppen, Carboxylgruppen, Aminogruppen, Nitrilfunktionen, Schwefelhaltige Substituenten, Estergruppen und/oder Ethergruppen substituiert sein können.

Die genannten Thiazole können sowohl als freie Base wie auch als Salz vorliegen: z. B. als Fluorid, Chlorid, Bromid, lodid, Sulfat, Carbonat, Ascorbat, Acetat oder Phoshat. Im Besonderen als Halogensalze, wie z. B. Chlorid und Bromid.

Weiterhin besteht eine vorteilhafte Verwirklichung der vorliegenden Erfindung in kosmetischen oder dermatologischen Zubereitungen mit einem wirksamen Gehalt an einem oder mehreren vorbenannten Alkylamidothiazolen.

Erfindungsgemäß ist ferner die Verwendung der vorgenannten Alkylamidothiazole zur Behandlung und/oder Prophylaxe unerwünschter Hautpigmentierung.

Dabei können Behandlung und/oder Prophylaxe unerwünschter Hautpigmentierung sowohl im kosmetischen wie im pharmazeutischen Rahmen erfolgen.

Dabei wird die pharmazeutische (oder dermatologische) Behandlung in erster Linie bei krankhaften Hautzuständen verstanden, wogegen die kosmetische Behandlung und/oder Prophylaxe unerwünschter Hautpigmentierung in erster Linie die gesunde Haut betrifft.

Vorteilhaft wird **X** gewählt aus der Gruppe der substituierten Phenyle, wobei die Substituenten (Z) gewählt werden können aus der Gruppe -H, -OH, -F, -Cl, -Br, -I, -OMe, -NH₂, -CN, Acetyl und gleich oder unterschiedlich sein können.

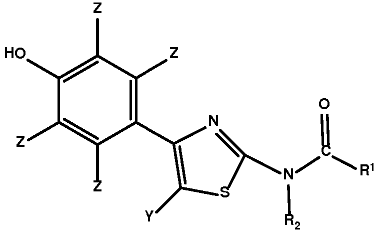

Besonders vorteilhaft wird X aus der Gruppe der mit einer oder mehreren Hydroxygruppen substituierten Phenylgruppen gewählt, wobei der Substituent (Z) gewählt werden kann aus der Gruppe -H, -OH, -F, -Cl, -Br, -I, -OMe, -NH₂, -CN, Acetyl und die folgende generische Struktur bevorzugt wird, bei welcher Y, R¹ und R² die vorstehend definierten Eigenschaften haben können.

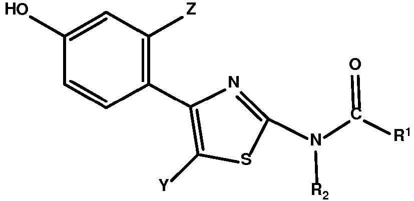

Vorteilhaft sind insbesondere solche Verbindungen, bei welchen
**X=** 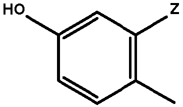
**Y** = H
**R₁** = -C₁-C₂₄-Alkyl (linear und verzweigt), -C₁-C₂₄-Alkenyl (linear und verzweigt), -C₁-C₈-Cycloalkyl, -C₁-C₈-Cycloalkyl-Alkylhydroxy, -C₁-C₂₄ Alkylhydroxy (linear und verzweigt), -C₁-C₂₄ Alkylamin (linear und verzweigt), -C₁-C₂₄-Alkylaryl (linear und verzweigt), -C₁-C₂₄-Alkylaryl-Alkyl-Hydroxy (linear und verzweigt), -C₁-C₂₄-Alkylheteroaryl (linear und verzweigt), -C₁-C₂₄-Alkyl-O-C₁-C₂₄-Alkyl (linear und verzweigt), -C₁-C₂₄ Alky-Morpholino, -C₁-C₂₄ Alky-Piperidino, -C₁-C₂₄ Alky-Piperazino, -C₁-C₂₄ Alky-Piperazino-N-Alkyl bedeutet,
**R₂** = H, -C₁-C₂₄-Alkyl (linear und verzweigt).
**Z** = -H, -OH, -F, -Cl, -Br, -I, -OMe, -NH₂, -CN, Acetyl.

Besonders bevorzugt sind solche Verbindungen, bei welchen
**X =** 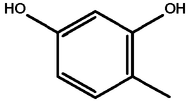
**Y** = H
**R₁** = -C₁-C₂₄-Alkyl (linear und verzweigt), -C₁-C₂₄-Alkenyl (linear und verzweigt), -C₁-C₈-Cycloalkyl, -C₁-C₈-Cycloalkyl-Alkylhydroxy, -C₁-C₂₄ Alkylhydroxy (linear und verzweigt), -C₁-C₂₄ Alkylamin (linear und verzweigt), -C₁-C₂₄-Alkylaryl (linear und verzweigt), -C₁-C₂₄-Alkylaryl-Alkyl-Hydroxy (linear und verzweigt), -C₁-C₂₄-Alkylheteroaryl (linear und verzweigt), -C₁-C₂₄-Alkyl-O-C₁-C₂₄-Alkyl (linear und verzweigt), -C₁-C₂₄ Alky-Morpholino, -C₁-C₂₄ Alky-Piperidino, -C₁-C₂₄ Alky-Piperazino, -C₁-C₂₄ Alky-Piperazino-N-Alkyl bedeutet,
**R₂** = H.

Die Verbindungen 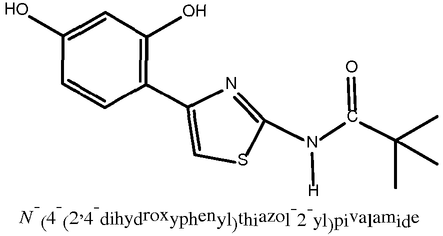 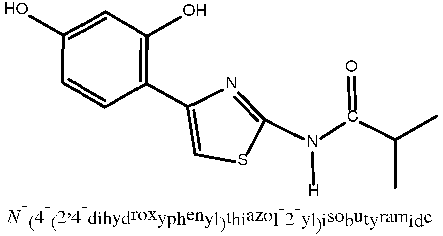 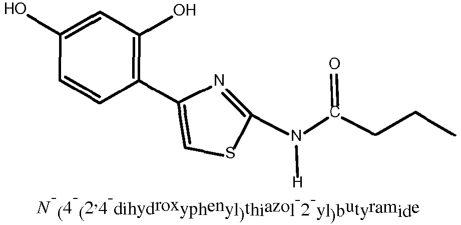 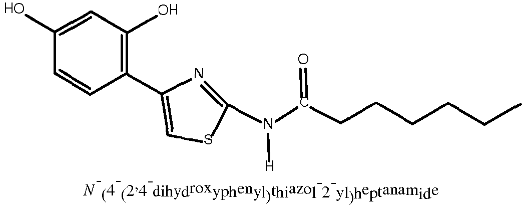 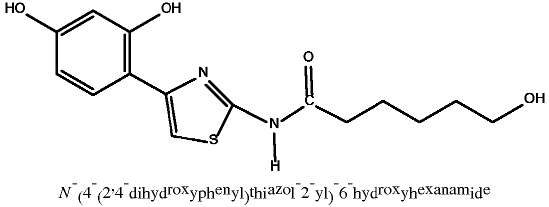 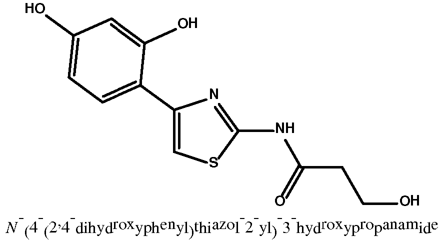 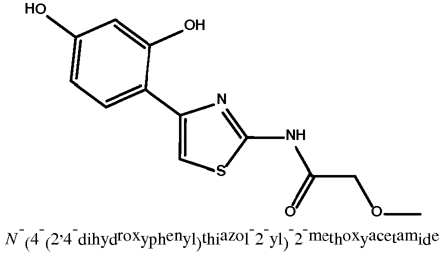 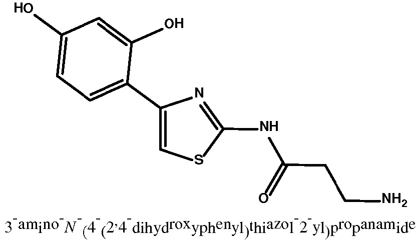 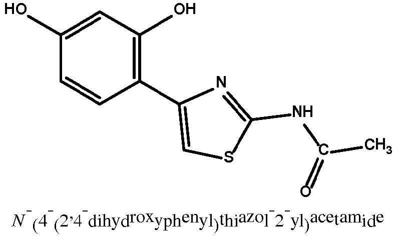 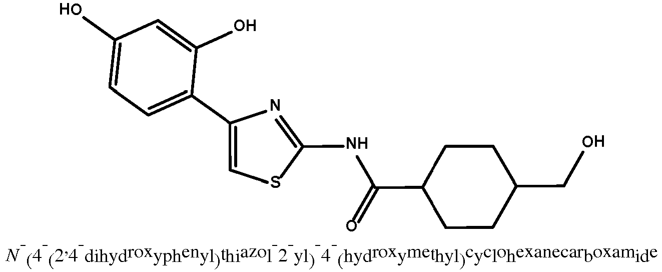 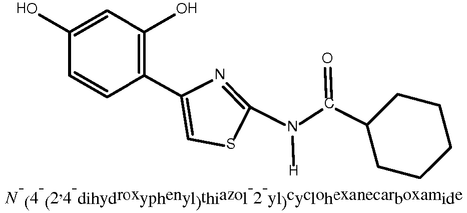 und 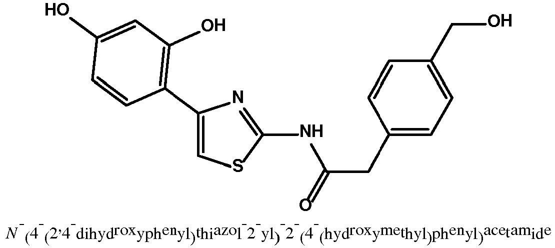 sind die erfindungsgemäß bevorzugten.

Zur Erarbeitung eines wirksamen selbstklebenden Pflasters mit Alkylamidothiazolen enthaltend mit optimierter Freisetzung zur Behandlung von Pigmentstörungen der Haut wurden unterschiedliche selbstklebende Matrixsysteme mit 1 % des Wirkstoffes hergestellt und auf ihre Freisetzungseigenschaften nach 24 Stunden auf Schweinehaut mittels Franzscher Zellen untersucht:
- Eine unpolare Matrix auf Basis von Synthese- und Naturkautschuk (KA)
- Ein polarer feucht klebender Film auf Basis Polyacrylsäure / Polyvinylalkohol (FKF)
- Eine unpolare Matrix auf Basis eines Polyacrylsäurecopolymers (PAC)
- Eine polare wasserfreie Gelmatrix auf Basis Polyacrylsäure / Polyvinylpyrrolidon (WFG)
- Eine unpolare Polyisobutylenmatrix (PIB)
- Ein polare Wassergelmatrix auf Basis Agar Agar / Polyacrylsäure (WG)

Neben den Liberationen im SEM wurden ebenfalls manuelle Liberationen in modifizierten Franz-Zellen durchgeführt.

Dieses in vitro-Modell dient ebenfalls zur Abschätzung der Liberation von Wirk- und Hilfsstoffen von zur topischen Applikation entwickelten Kosmetika. Es eignet sich genauso zum schnellen Vergleich von unterschiedlichen Formulierungen und eine Kinetik über frei gewählte Zeitpunkte kann aufgenommen werden.

Die Versuche wurden ebenfalls mit einer Nitrocellulose-Membran als Barriere zwischen Prüfmuster und Rezeptorphase durchgeführt. Die Rezeptorphase kann frei gewählt werden. Bei der Durchführung in bis zu 33 nahezu identischen Zellen erhält man den Verlauf der Liberation zu den gewählten Zeitpunkten, durch den analytischen Nachweis der Prüfsubstanz in der Rezeptorphase. Vor der Verwendung wurden die Franz-Zellen mit Isopropanol, Gazen und Pinzetten gereinigt.

Je Prüfmuster wurden 3 Zellen benötigt. Jede Zelle wurde mit ca. 11 ml Rezeptorphase gefüllt (Blasenfrei bis kurz über den Rand). Für jede Zelle wurde je Probennahme ein offenes Vial vorbereitet. Die Zeitpunkte der Probennahme können frei gewählt werden und hängen von der Liberationsgeschwindigkeit der Prüfsubstanz in die Rezeptorphase ab. Diese muss zuvor z. B. durch einschlägige Literatur eingeschätzt oder einfach ausprobiert werden. Die Liberation lief mit Temperierung der Zellen mittels Doppelmantel und Wasserbad auf ca. 32°C. Die Rezeptorphase wurde mit 600 rpm und Magnetrührstäbchen mit Hilfe der Rührplatte unter den Zellen gerührt.

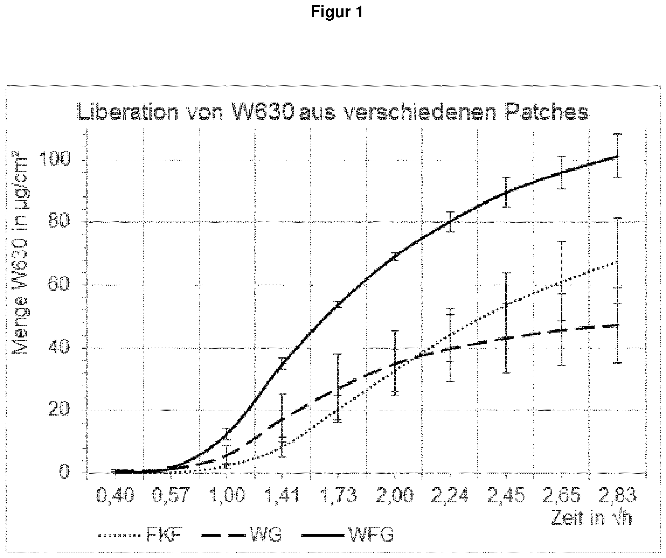

Die Prüfmuster wurden auf die angefeuchtete Membran aufgebracht. Die Creme wurde dafür mit einem Spatel auf einer Fläche von 4,16 cm² aufgetragen. Die Patches mit einer Fläche von 4,16 cm² (Ø = 2,3 cm²) wurden auf der Membran aufgebracht. Die angefeuchtete Membran mit dem Prüfmuster drauf wurde unter Benutzung von zwei Pinzetten plan und luftblasenfrei auf die gefüllten Zellen gelegt und mit dem Deckel befestigt. Der genaue Aufbau der Franz-Zellen kann der Abbildung 8 entnommen werden. Das Probennahme-Volumen ist auf 1 ml festgelegt. Die Probennahme erfolgte manuell mittels Spritzen nach dem Abnehmen der Prüfsubstanz zusammen mit der Membran. Das Abnehmen der Membran erfolgte ebenfalls mit zwei Pinzetten nachdem der Deckel abgenommen wurde. Mit der Membran nach unten wurde sie zusammen mit dem Prüfmuster auf ein Trennpapier gelegt. Die Proben wurden durch einen Spritzenvorsatzfilter mit 0,2 µm filtriert und direkt in ein Vial gegeben. Das Vial wurde mit Bördelkappe und Zange verschlossen und so schnell wie möglich in der HPLC vermessen. Die Proben wurden für die weitere Analytik nicht verdünnt und konnten ohne umfüllen in die HPLC gestellt werden. Proben für W630 und AGR wurden zu folgenden Zeitpunkten entnommen: 1 h, 2 h, 3 h, 5 h, 7 h und 24 h nach dem jeweiligen aufbringen des Patches. Proben für Q10 wurden zu folgenden Zeitpunkten entnommen: 1 h, 2 h, 3 h, 5 h und 7 h nach dem jeweiligen aufbringen des Patches. Nach jeder Probennahme erfolgte das Auffüllen der Zelle mit Rezeptorphase. Der Zeitaufwand von ein bis zwei Minuten zwischen dem Herunternehmen und dem erneuten Applizieren des Prüfmusters zu den Probennahmen muss bei den Zeitpunkten berücksichtigt werden. Deswegen wurden zu Beginn der Liberation alle Prüfmuster in einem Abstand von mindestens zwei Minuten auf die Zellen gelegt und der jeweilige Zeitpunkt zu den einzelnen Proben notiert. Daraus ergaben sich die jeweiligen Probennahme-Zeitpunkte. (Fig. 1).

### Ergebnisse der Wirkstofffreisetzung unterschiedlicher Matrixsysteme:

| (KA) | (FKF) | (PAC) | (WFG) | (PIB) | (WG) |
|---|---|---|---|---|---|
| 7,4 % | 17,8 % | 18,1 % | 20,6 % | 22,2 % | 34,0 % |

Basierend auf obigen ersten Ergebnissen wurden die polare Wassergelmatrix für weitere Optimierungsuntersuchungen hinsichtlich der Freisetzung von einem oder mehreren Alkylamidothiazole ausgewählt.

Die Wirkstofffreisetzung kann vorteilhaft durch die Schichtdicke des TTS gesteuert werden.

Es wurden von beiden Systemen, PIB und WG, jeweils Muster mit 1,00; 0,75; 0,50; 0,30 und 0,15 mm Schichtdicke hergestellt und untersucht.

### Ergebnisse der Wirkstofffreisetzung unterschiedlicher Matrixschichtdicken:

| | 1,00 mm | 0,75 mm | 0,50 mm | 0,30 mm | 0,15 mm |
|---|---|---|---|---|---|
| (PIB) | 22,2 % | 27,4 % | 26,7 % | 42,7 % | 46,4 % |
| (WG) | 34,0 % | 48,3 % | 41,8 % | 66,2 % | 56,8 % |

Als Kompromiss zwischen prozentualer Wirkstofffreisetzung, absoluter freigesetzter Wirkstoffmenge und Handhabbarkeit des fertigen selbstklebenden Pflasters wird bei beiden Matrixsystemen eine Schichtdicke von 0,30 mm erachtet. Insbesondere das selbstklebende Patch basierend auf einem erfindungsgemäßen polaren Wassergel zeigt mit rund 65 % des Wirkstoffs eine im Vergleich zu üblichen Pflasteranwendungen überraschend hohe Freisetzung.

Um das PIB-Matrixsystem ebenfalls in diesen Bereich der Wirkstofffreisetzung zu bringen wurden weitere Versuche mittels Einarbeitung von üblichen Additiven zur Erhöhung der Hydrophile der Matrix sowie auch von üblichen Penetrationsbeschleunigern durchgeführt.

Die Einarbeitung von 35 % Cellulose in die PIB Matrix ergab keinen signifikanten Unterschied in der Wirkstofffreisetzung gegenüber einer nicht mit Cellulose gefüllten Matrix.

Als übliche Penetrationsbeschleuniger wurden dann jeweils 5 % Isopropylpalmitat (IPP) bzw. 5 % Isopropylmyristat (IPM) in eine entsprechende PIB-Matrix eingearbeitet und die Freisetzung an ein oder mehrere Alkylamidothiazole aus den daraus hergestellten Endprodukten von 0,30 mm Schichtdicke bestimmt.

Beispiele für die Herstellung polarer selbstklebender Wassergel-Matrices sind in DE 102 60 872 beschrieben.

Beispiele für die Herstellung unpolarer selbstklebender PIB-Matrices sind in EP 1 335 755 beschrieben.

Beispiele für die Herstellung von wasserfreien Gelen sind in EP 1 430 098 beschrieben.

Als Trägermaterialien für erfindungsgemäße polare wie unpolare selbstklebende Patches mit optimierter Wirkstofffreisetzung zur Behandlung von Pigmentstörungen der Haut eignen sich alle gängigen flächigen Schichtmaterialien wie beispielsweise Gewebe, Folien, Vliese etc. Besonders vorteilhaft ist die Verwendung von sehr dünnen, Schichtdicke unter 100 µm, flexiblen und durchsichtigen Polymerfolien, insbesondere solchen aus wässriger Dispersion hergestellten Polyurethanfolien.

Da die erfindungsgemäßen Wassergel- wie auch wasserfreie Gele transparent bis maximal translucent sind, werden mit den vorstehend beschriebenen Folien als Trägermaterialien kaschierten Patches optisch kaum wahrgenommen und können daher auch über längere Zeiträume unauffällig angewendet werden. Durch eine entsprechende Einfärbung der Matrices oder Abdeckung mit einem vorgefärbten Träger kann ein erfindungsgemäßes Patch aber auch in unauffälliger Hauttönung hergestellt werden.

Erfindungsgemäße selbstklebende Patches können jedwede beliebige Form und Größe haben, z. B. rund, rechteckig, quadratisch etc. Besonders vorteilhaft ist, das erfindungsgemäße Patches auf WFG wie auch auf WG-Basis vom Anwender mittels handelsüblicher Scheren in jedwede beliebige Form und Größe zu Recht geschnitten werden können, um das Patch dem gewünschten Behandlungsareal exakt anzupassen.

Die folgenden Beispiele sollen die Erfindung erläutern.

### Beispiel 1: Feuchtklebender Film

| **dass** | **inci** | **Gew.-%** |
|---|---|---|
| Rheological modifier | Polyvinyl Alcohol | 33,5 |
| Solvent | Aqua | 49,7 |
| Rheological modifier | Acrylates/C10-30 Alkyl Acrylate Crosspolymer | 4 |
| Rheological modifier | Sodium Polyacrylate | 4 |
| Moisturiser | Propylene Glycol | 8,5 |
| Active | Isobutylamido Thiazolyl Resorcinol | 0,3 |

### Beispiel 2: Wasserfreies Gel

| **dass** | **inci** | **Gew.-%** |
|---|---|---|
| Moisturiser | Propylene Glycol | 5 |
| Active | Isobutylamido Thiazolyl Resorcinol | 0,3 |
| Moisturiser | Glycerin | 40,7 |
| Active | Panthenol | 3 |
| Solvent | PEG-8 + Aqua | 18,5 |
| Rheological modifier | Carbomer | 22 |
| Film former | PVP | 3,5 |
| Filler | Silica | 7 |

### Beispiel 3: Wassergel

| **dass** | **inci** | **Gew.-%** |
|---|---|---|
| Solvent | Aqua | 47,2 |
| Moisturiser | Propylene Glycol | 2,5 |
| Active | Isobutylamido Thiazolyl Resorcinol | 0,3 |
| Moisturiser | Glycerin | 17 |
| Solvent | PEG-8 + Aqua | 5 |
| Rheological modifier | Carbomer | 8 |
| | Agar | 2 |
| Moisturiser | Sorbitol + Aqua | 15 |
| Neutralising agent | Aqua + Sodium Hydroxide | 3 |

## Patentansprüche

1. Selbstklebende flächige Gebilde, insbesondere kosmetische und/oder medizinische Pflaster, welche als Wirkstoff ein oder mehrere Alkylamidothiazole enthalten, **dadurch gekennzeichnet, dass** sie als Matrixsysteme vorliegen und die Matrix gewählt wird aus den unpolaren Matrices auf Basis von Synthese- und Naturkautschuk (KA) dem polaren feucht klebenden Film auf Basis von Polyacrylsäure / Polyvinylalkohol (FKF) den unpolaren Matrices auf Basis von Polyacrylsäurecopolymeren (PAC), den polaren wasserfreien Gelmatrices auf Basis Polyacrylsäure / Polyvinylpyrrolidon (WFG) den unpolaren Polyisobutylenmatrices (PIB), den polaren Wassergelmatrices auf Basis Agar Agar / Polyacrylsäure (WG).

2. Flächige Gebilde nach Anspruch 1, **dadurch gekennzeichnet, dass** sie 0,001 - 10 Gew.-%, besonders bevorzugt 0,01 - 1 Gew.-%, an einem oder mehreren Alkylamidothiazole enthalten, bezogen auf die Gesamtzusammensetzung der Zubereitungen.

3. Flächige Gebilde nach einem der vorstehenden Ansprüche, **dadurch gekennzeichnet, dass** als Lösungsvermittler für das Alkylamidothiazol Propylenglycol, Ethanol, Butylenglycol eingesetzt wird.

4. Flächige Gebilde nach einem der vorstehenden Ansprüche, **dadurch gekennzeichnet, dass** die Zubereitungen 0,001 - 10 Gew.-%, besonders bevorzugt 0,01 - 5 Gew.-%, ganz besonders bevorzugt 0,1 - 1 Gew.-% an Penetrationsbeschleuniger, insbesonde re Isopropylpalmitat und/oder Isopropylmyristat.enthalten, bezogen auf die Gesamtzu sammensetzung der Zubereitungen.

5. Flächige Gebilde nach einem der vorstehenden Ansprüche, **dadurch gekennzeichnet, dass** als Alkylamidothiazol die Substanz der Formel 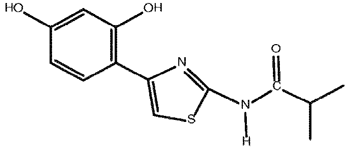 gewählt wird.

## Claims

1. Self-adhesive sheet-like structures, in particular cosmetic and/or medical plasters, which comprise one or more alkylamidothiazoles as active ingredient, **characterized in that** they are in the form of matrix systems and the matrix is selected from the nonpolar matrices based on synthetic and natural rubber (KA), the polar wet-adhesive film based on polyacrylic acid/polyvinyl alcohol (FKF), the nonpolar matrices based on polyacrylic acid copolymers (PAC), the polar anhydrous gel matrices based on polyacrylic acid/polyvinylpyrrolidone (WFG), the nonpolar polyisobutylene matrices (PIB), the polar water gel matrices based on agar agar/polyacrylic acid (WG).

2. Sheet-like structures according to Claim 1, **characterized in that** they comprise 0.001% - 10% by weight, particularly preferably 0.01% - 1% by weight, of one or more alkylamidothiazoles, based on the total composition of the preparations.

3. Sheet-like structures according to either of the preceding claims, **characterized in that** propylene glycol, ethanol or butylene glycol is used as solubilizer for the alkylamidothiazole.

4. Sheet-like structures according to any of the preceding claims, **characterized in that** the preparations comprise 0.001% - 10% by weight, particularly preferably 0.01% - 5% by weight, very particularly preferably 0.1% - 1% by weight, of penetration accelerator, in particular isopropyl palmitate and/or isopropyl myristate, based on the total composition of the preparations.

5. Sheet-like structures according to any of the preceding claims, **characterized in that** the substance of the formula 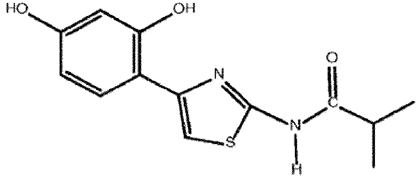 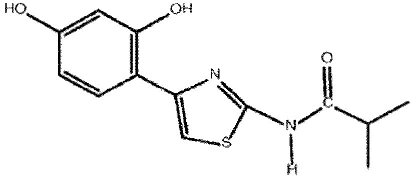 is selected as alkylamidothiazole.

## Revendications

1. Produits plats autoadhésifs, en particulier timbres transdermiques cosmétiques et/ou médicaux, qui contiennent en tant que principe actif un ou plusieurs alkylamidothiazoles, **caractérisés en ce qu'**ils se présentent sous forme de systèmes de matrice, et la matrice est choisie parmi les matrices apolaires à base de caoutchouc synthétique et naturel (KA), un film polaire adhésif par voie humide à base de poly(acide acrylique / poly(alcool vinylique) (FKF), les matrices apolaires à base de copolymères de poly(acide acrylique) (PAC), les matrices en gel anhydres polaires à base de poly(acide acrylique) / polyvinylpyrrolidone (WFG), les matrices de polyisobutylène (PIB) apolaires, les matrices en gel aqueux polaires à base d'agar agar/poly(acide acrylique) (WG).

2. Produits plats selon la revendication 1, **caractérisés en ce qu'**ils contiennent 0,001 - 10 % en poids, en particulier 0,01 - 1 % en poids d'un ou plusieurs alkylamidothiazoles, par rapport à la composition totale des préparations.

3. Produits plats selon l'une des revendications précédentes, **caractérisés en ce qu'**on utilise comme solubilisant pour l'alkylamidothiazole du propylèneglycol, de l'éthanol, du butylèneglycol.

4. Produits plats selon l'une des revendications précédentes, **caractérisés en ce que** les préparations contiennent 0,001 - 10 % en poids, d'une manière particulièrement préférée 0,01 - 5 % en poids, d'une manière tout particulièrement préférée 0,1 - 1 % en poids d'un accélérateur de pénétration, en particulier le palmitate d'isopropyle et/ou le myristate d'isopropyle, par rapport à la composition totale des préparations.

5. Produits plats selon l'une des revendications précédentes, **caractérisés en ce qu'**on choisit comme alkylamidothiazole la substance de formule
